(19) Europäisches Patentamt / European Patent Office / Office européen des brevets

(11) **EP 3 981 443 A1**

(12) **EUROPEAN PATENT APPLICATION**

(43) Date of publication:
**13.04.2022 Bulletin 2022/15**

(21) Application number: **20200785.2**

(22) Date of filing: **08.10.2020**

(51) International Patent Classification (IPC):
**A61L 15/32** *(2006.01)*        **A61L 15/44** *(2006.01)*

(52) Cooperative Patent Classification (CPC):
**A61L 15/32; A61L 15/44;** A61L 2300/252;
A61L 2300/412

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Johann Wolfgang Goethe-Universität Frankfurt**
**60323 Frankfurt am Main (DE)**

(72) Inventors:
• **Windbergs, Maike**
  **61462 Königstein im Taunus (DE)**
• **Walther, Marcel**
  **60433 Frankfurt am Main (DE)**
• **Planz, Viktoria**
  **60439 Frankfurt am Main (DE)**

(74) Representative: **Krauss, Jan**
**Boehmert & Boehmert**
**Anwaltspartnerschaft mbB**
**Pettenkoferstrasse 22**
**80336 München (DE)**

(54) **IMPROVED WOUND DRESSINGS, METHODS FOR MAKING THESE, AND USES THEREOF**

(57)    The present invention relates to core-shell fibers based on biocompatible polymers incorporating proteohormones with growth factor properties, methods of making these fibers, and respective uses of these fibers in effective wound therapy systems.

EP 3 981 443 A1

**Description**

The present invention relates to core-shell fibers based on biocompatible polymers incorporating proteohormones with growth factor properties, methods of making these fibers, and respective uses of these fibers in effective wound therapy systems.

Background of the invention

Despite significant research efforts, wound healing still represents a major medical challenge, further impaired by chronicity and invasion of infectious pathogens. Current therapies are far away from assuring adequate healing, resulting in frequent relapses, which account for morbidity and mortality of numerous patients as well as for a substantial economic burden.

US 8,728,498 ("Electrospun silk material systems for wound healing") relates to silk fibroin/polyethylene oxide materials and their use in wound healing. The materials can include active compounds that improve wound healing and can be released over a period of time, such as, for example, antibiotics, hormones, peptides, growth factors (e.g. VEGF, EGF, and IGF-I), and the like.

KR 2008-0104932A ("Nanofiber nonwoven comprising chitosan, biodegradable polymer and cell growth factor and method of preparing thereof") discloses a nanofiber, electrospun, non-woven fabric containing chitosan, a biodegradable polymer mainly consisting of chitosan fibers. The example exhibits that insulin can be embedded in the fibers.

US 9,744,262 ("Alimentary protein-based scaffolds (APS) for wound healing, regenerative medicine and drug discovery") discloses an electrospun composition comprising fibers of plant product derived biomaterials. The biomaterial can be electroprocessed to produce fibers, and the scaffold thereof may comprise an agent, such as a growth factor, such as insulin.

US 7,041,868 ("Multi-Layered Antiadhesion Barrier") discloses a scaffold material that can be impregnated with bioactive agents such as drugs, vitamins, growth factors, therapeutic peptides, and the like.

CN 107397973 ("A kind of four layers of coaxial fiber wound dressing and preparation method thereof') discloses four-layered coaxial fiber wound dressings. The material can be prepared by a coaxial electrospinning process.

Wei et al. (in: Wei, Q., Xu, F., Xu, X. et al. The multifunctional wound dressing with core-shell structured fibers prepared by coaxial electrospinning. Front. Mater. Sci. 10, 113-121 (2016). https://doi.org/10.1007/s11706-016-0339-7) discloses a non-woven wound dressing with core-shell structured fibers that was prepared by coaxial electrospinning. Polycaprolactone (PCL) was electrospun as the fiber's core to provide mechanical strength, whereas collagen was fabricated into the shell in order to utilize its good biocompatibility. Simultaneously, silver nanoparticles (Ag-NPs) as anti-bacterial agent were loaded in the shell, whereas vitamin A palmitate (VA) as healing-promoting drug was encapsulated in the core. The dressing's anti-bacteria ability against *Staphylococcus aureus* was proved by *in vitro* anti-bacteria test.

Mulholland EJ. (in: Electrospun Biomaterials in the Treatment and Prevention of Scars in Skin Wound Healing. Front Bioeng Biotechnol. 2020;8:481. Published 2020 Jun 3. doi:10.3389/fbioe.2020.00481) discusses the molecular processes of wound healing and scar tissue formation, the process of electrospinning, and examine how electrospun biomaterials can be utilized and adapted to wound repair in the hope of reducing scar tissue formation and conferring an enhanced tensile strength of the skin.

Topical application of growth factors, the natural key players in wound healing processes, bares a high potential for effective wound therapy, but is impeded by rapid degradation of the actives and a lack of suitable delivery systems.

It is therefore an object of the present invention to provide methods and materials for the production of improved materials for delivery systems in effective wound therapy, as well as respective delivery systems. Other objects and advantages will become apparent to the person of skill when studying the present description of the present invention.

In a first aspect of the present invention, this object is solved by providing a method for producing core-shell fibers of biocompatible polymers comprising a bioactive proteohormone, comprising the steps of a) providing a suitable shell biocompatible polymer solution, b) providing a suitable core solution of said bioactive proteohormone, and c) coaxial electrospinning of said shell biocompatible polymer solution and said core solution of said bioactive proteohormone in order to produce said core-shell fibers. Preferred is the method according to the present invention, wherein said bioactive proteohormone is selected from hormones involved in wound healing, human growth hormone (HGH), insulin-like growth factor-1 (IGF-1), insulin, testosterone, dehydroepiandrosterone (DHEA), and estrogen.

In a second aspect of the present invention, the above object is solved by providing coaxial core-shell fibers of biocompatible polymers comprising a bioactive proteohormone, produced according to the method according to the present invention.

In a third aspect of the present invention, the above object is solved by providing a patch, fibrous membrane, dressing or gauze for use with wounds, comprising coaxial core-shell fibers according to the present invention.

Another aspect of the present invention relates to the use of coaxial core-shell fibers according to the present invention in wound therapy systems.

Yet another aspect of the present invention then relates to a bioactive proteohormone for use in the therapy of wounds, comprising administering said proteohormone using the patch, fibrous membrane, dressing or gauze according to the present invention.

In the context of the present invention, the present inventors developed an improved method for producing core-shell fibers of biocompatible polymers comprising a bioactive proteohormone. The present invention comprises the steps of a) providing a suitable shell biocompatible polymer solution, b) providing a suitable core solution of said bioactive proteohormone, and c) coaxial electrospinning of said shell biocompatible polymer solution and said core solution of said bioactive proteohormone in order to produce said core-shell fibers.

Wounds are a severe pathologic condition with yet unmet therapeutic needs, imposing not only health risks, but also causing a high socio-economical healthcare burden. The treatment cost associated with hospitalization, dressing changes and associated bacterial infections are estimated 6.000-10.000 € per patient and year.[1] Furthermore, the number of patients suffering from wounds aggravating towards chronicity has dramatically increased, currently affecting 1-2 % of the European and United States population.[2,3] Current treatment strategies for wounds still mostly focus on wound dressings, providing a moist environment supplemented with application of anti-inflammatory actives and/or antiseptics. Despite technologic advances in material sciences and significant interdisciplinary research efforts, effective medical treatment options are scarce.

Since the formation of new tissue and thus wound closure is regulated by several growth factors, cytokines and chemokines, the administration of growth factors for wound repair has gained significant interest. As chronic wounds are additionally characterized by reduced growth factor levels in the wound bed, supplementation of such molecules into the wound bed seems a logical consequence to promote wound healing.[4,5] Apart from well-known growth factors, such as epidermal growth factor and fibroblast growth factor, which at the same time exhibit an oncogenic potential, proteohormes, such as the peptide hormone insulin, are catching increasing attention in the field of wound healing.[6,7] Insulin, for example, is known for its stimulating and proliferative effects within human tissue, acting as a growth factor, with the advantage of low oncogenic potential over the above mentioned.

However, the therapeutic application of such biomolecules for wound healing is challenging, requiring delivery in a localized and controlled manner, while ensuring the stability of the molecule during processing.[8,9] Growth factors are usually administered in solution or using semisolid formulations such as creams, gels or ointments.[10] However, none of these formulations provides controlled release of the biomolecules and thus requires continuous administration or high doses to maintain therapeutic concentrations in the wound bed. Further, undesired side effects can occur due to high systemic availability of the growth factors, limiting their clinical application.

In this context, electrospun fibers offer unmet possibilities as drug delivery systems with controlled-release properties.[11-13] Incorporation of active compounds, such as the proteohormones as herein, into such fibers not only protects the fragile molecules, but also provides release in a controlled manner and in direct proximity to the wound bed, when applied as wound dressings. Further, such dressings are able to protect the wound against external environmental factors and bacterial contamination, and establish a moist wound climate, while allowing gas exchange.[14,15]

In order to resolve the above mentioned problems, the inventors developed methods to produce improved proteohormone-loaded, in particular insulin-loaded core-shell fibers, and respective innovative dual-function wound dressings, capable of overcoming the challenges of current wound therapy. As a proof of concept, the inventors have shown that insulin, a peptide hormone acting as a growth factor, accelerates wound healing of human skin cells. With the encapsulation of insulin into the core of coaxially electrospun fibers, insulin is not only stabilized in its active form, but at the same time its controlled delivery directly into the wound bed is ensured. The wound dressing was designed from biocompatible polymers, featuring optimal mechanical protection and flexibility, whilst providing a moist wound healing climate and allowing gas exchange. Wound healing related proteohormone-loaded (e.g. insulin-loaded) fibers thus show a high potential to become the next generation wound dressings in the near future, solving current challenges in wound therapy.

Preferred is the method according to the present invention, wherein said bioactive proteohormone is selected from hormones involved in wound healing, human growth hormone (HGH), insulin-like growth factor-1 (IGF-1), insulin, testosterone, dehydroepiandrosterone (DHEA), and estrogen. Preferred is insulin, for example human insulin, such as recombinantly produced insulin.

Preferred is the method according to the present invention, wherein said suitable core solution comprises a suitable slightly acidic buffer system, preferably comprising phosphate buffered saline (PBS), glycerol and hydrochloric acid. Preferred is a slightly acidic pH, for example at about between 5.5 and 7.0.

In the context of the present invention, the term "about" shall mean a deviation of +/- 10% from the value as given, unless indicated otherwise.

In yet another aspect of the method according to the present invention the core solution may further comprises suitable additional pharmaceutically active compounds, such as, for example, antimicrobial agents, such as antibacterial and antifungal agents, and/or silver particles. Of course, these compounds need to be compatible with the proteohormone (e.g. the function and/or stability thereof), the core solution components (e.g. the buffer system), and the production method (electrospinning).

In yet another aspect of the method according to the present invention the biocompatible polymer is suitable for biomedical applications, in particular for use in wound healing. Factors to be considered for use of a biocompatible polymer, particularly for the shell of the fiber, include and are based on biocompatibility, hydrophilicity, hydrophobicity, dissolution, solubility, and/or scaffold stability of the polymer, as well as the final shell material as used. Of course, the material must be suitable for elctrospinning. Preferred are biocompatible polymers that can act as hydrogen bond donors, facilitate the interaction with water, and lead to water absorption, and/or also retain structural integrity. The surface of fibers as produced in the examples below was found to be quite smooth, and small voids have formed, most likely due to the dissolution of the water soluble polymer PEO, thus preferably facilitating the release of active compounds, such as insulin.

Examples of preferred biocompatible polymers are selected from polycaprolactone (PCL), chitosan, alginate, polyethylene oxide (PEO)/polyethylene glycol (PEG), and/or poly(D,L-lactic-co-glycolic) acid (PLGA) and biocompatible derivatives thereof. These derivatives are known to the person of skill, and can be taken from the respective literature (e.g. for PLGA, see Sah H, Thoma LA, Desu HR, Sah E, Wood GC. Concepts and practices used to develop functional PLGA-based nanoparticulate systems. Int J Nanomedicine. 2013;8:747-765. doi:10.2147/IJN.S40579; Snejdrova E, Podzimek S, Martiska J, Holas O, Dittrich M. Branched PLGA derivatives with tailored drug delivery properties. Acta Pharm. 2020;70(1):63-75. doi:10.2478/acph-2020-0011

Khil et al. (in: Electrospun nanofibrous polyurethane membrane as wound dressing. J Biomed Mater Res B Appl Biomater. 2003;67(2):675-679. doi:10.1002/jbm.b.10058) describe a polyurethane membrane produced via electrospinning. The porous structured electrospun membrane was found to have favorable properties: it exudated fluid from the wound, did not build up under the covering, and did not cause wound desiccation. The electrospun nanofibrous membrane showed controlled evaporative water loss, excellent oxygen permeability, and promoted fluid drainage ability, but still could inhibit exogenous microorganism invasion because its pores are ultra-fine. The electrospun membrane was disclosed as potential application for wound dressings based upon its unique properties.

Preferred is further a method according to the present invention, wherein a mix or blend of biocompatible polymers is used for the shell, thus, wherein said biocompatible polymer solution comprises at least two biocompatible polymers. These polymers need to be compatible for the purpose of creating coaxial fibers, and preferably are compatible as indicated by one glass transition temperature after a first heating. This can be done using standard methods, like Differential Scanning Calorimetry (DSC), or Thermal Mechanical Analysis (TMA).

Furthermore, the biopolymer blends must also be compatible with the proteohormone(s) to be used in the fibers and patches. Chou and Woodrow (in: Relationships between mechanical properties and drug release from electrospun fibers of PCL and PLGA blends. J Mech Behav Biomed Mater. 2017;65:724-733. doi:10.1016/j.jmbbm.2016.09.004) investigated the mechanical properties of electrospun polycaprolactone (PCL) and poly (D,L-lactic-co-glycolic) acid (PLGA) fibers at various blend ratios in the presence and absence of a small molecule hydrophilic drug, tenofovir (TFV).

In the context of the method according to the present invention, any suitable solvent may be used to dissolve the biocompatible polymer(s) for the shell solution. The solvent needs to be compatible with the biocompatible polymer(s), and must be suitable for the electrospinning process. Also a mixture of suitable solvents can be used, such as, for example, alcohols, such as ethanol, and chloroform.

In a particularly preferred embodiment of the method according to the present invention, a suitable shell biocompatible polymer solution was provided with polyethylene oxide (PEO) and polycaprolactone (PCL), here PEO 600 kDa, 1.8% w/v, and PCL 80 kDa, 10.2% w/v, dissolved in ethanol and chloroform, preferably at a 1:1 v/v ratio. For the core solution, in order to obtain a suitable insulin concentration (e.g. of 11.5 mg/mL), 15 mg powdered insulin (human, recombinant) were dissolved in 750 $\mu$L PBS buffer, acidified with 25 $\mu$L HCl, and mixed with 530 $\mu$L glycerol (85%) under sterile conditions. Both solutions were connected to the shell or core port, respectively, of a coaxial spinning needle. Then, coaxial electrospinning was performed, at 7 kV with flow rates of 0.28 mL/h (core) and 2.8 mL/h (shell), onto a rotating metal drum collector (250 RPM), in order to produce the inventive core-shell fibers.

Another aspect of the present invention then relates to a coaxial core-shell fiber of biocompatible polymers comprising a bioactive proteohormone, produced according to the method according to the present invention as described herein. In the context of the invention, it was surprisingly found that in the coaxial core-shell fiber according to the present invention the bioactive proteohormone(s) is/are stabilized in the fiber core during storage and/or the release thereof into the wound site is prolonged (sustained release). Advantageously, for example, the surface morphology of said coaxial core-shell fibers changes after application, and small voids are formed. This allows the active compounds in the fiber to be released over time.

Yet another aspect of the present invention then relates to a patch, fibrous membrane, dressing or gauze for use with wounds or in wound healing, in particular a wound of or wound healing in a mammal, such as a human subject, comprising coaxial core-shell fibers according to the present invention. How to produce respective devices is disclosed herein, and furthermore known to the person of skill and can be found in the respective literature (see, for example, Wei et al., above).

Preferred is a patch, fibrous membrane, dressing or gauze for use with wounds or in wound healing according to the present invention that shows a fluid (water) retention of 400 $\pm$ 25% of the dry weight thereof. Further preferred is a patch, fibrous membrane, dressing or gauze for use with wounds or in wound healing according to the present invention, wherein suitable therapeutic concentrations of said bioactive proteohormone(s) are reached within 1 hour after application thereof, and are maintained for at least 24 hours.

It is particularly preferred that in the patch, fibrous membrane, dressing or gauze for use with wounds or in wound healing according to the present invention the surface morphology of said coaxial core-shell fibers changes after application in that small voids are formed in the individual fibers. This is particularly achieved with blends of polymers wherein one polymer is (at least) partially soluble in the wound environment, such as PEO, while the patch preferably retains its structural integrity due to insoluble components, such as PCL.

The patch, fibrous membrane, dressing or gauze for use with wounds or in wound healing according to the present invention is particularly biocompatible with the wound environment in during use cells involved in healing (e.g. keratinocytes and fibroblasts) can form a thin layer on the very surfaces thereof, but do not significantly invade into the patch, fibrous membrane, or gauze for use with wounds or wound dressing. Furthermore, said cells exhibit a healthy morphology, while showing an accelerated and improved wound healing process when treated with insulin.

Since the cells do not invade the patch, fibrous membrane, dressing or gauze for use with wounds or in wound healing according to the present invention the patch, fibrous membrane, dressing or gauze allows for a regular change of the dressing without fusion of wound and dressing (sticking), and without inflicting tissue damage when being removed. This further accelerates the healing and avoids or reduces scar formation.

Particularly preferred is the patch, fibrous membrane, dressing or gauze for use with wounds or in wound healing according to the present invention, wherein said wound is a chronic wound, for example a diabetic ulcer.

Another aspect of the present invention relates to the use of coaxial core-shell fibers according to the present invention in wound therapy systems, such as patches, fibrous membranes, dressings or gauzes as disclosed herein.

Another aspect of the present invention relates to a bioactive proteohormone for use in the therapy of wounds, or the promotion of wound healing in a mammalian patient, such as a human, comprising administering said proteohormone using the patch, fibrous membrane, dressing or gauze according to the present invention. Preferred is the bioactive proteohormone for use according to the present invention, wherein said proteohormone is selected from hormones involved in wound healing, human growth hormone (HGH), insulin-like growth factor-1 (IGF-1), insulin, testosterone, dehydroepiandrosterone (DHEA), and estrogen and combinations thereof. Further preferred is the use in combination with suitable additional pharmaceutically active compounds, such as, for example, antimicrobial agents, such as antibacterial agents, and/or silver particles. Particularly preferred is the bioactive proteohormone for use according to the present invention, wherein said wound is a chronic wound, for example a diabetic ulcer.

Another aspect of the present invention relates to a method of treating or preventing a wound or promoting wound healing in a mammalian patient, such as a human, comprising administering a proteohormone promoting wound healing using the patch, fibrous membrane, dressing or gauze according to the present invention to said patient in need thereof. Preferred is the method according to the present invention, wherein said proteohormone is selected from hormones involved in wound healing, human growth hormone (HGH), insulin-like growth factor-1 (IGF-1), insulin, testosterone, dehydroepiandrosterone (DHEA), and estrogen and combinations thereof. Further preferred is the method according to the present invention in combination with suitable additional pharmaceutically active compounds, such as, for example, antimicrobial agents, such as antibacterial agents, and/or silver particles. Particularly preferred is the method according to the present invention, wherein said wound is a chronic wound, for example a diabetic ulcer.

As mentioned above, in this invention, core-shell fibers based on biocompatible polymers (polycaprolactone/polyethylene oxide) were designed. In the core, insulin, a well-characterized proteohormone with growth factor properties, was incorporated, thus protecting this fragile molecule and controlling its release kinetics into the wound bed. The fibers were fabricated by electrospinning, subsequently forming wound dressings that exhibited optimal physicochemical properties for wound protection and establishment of a favorable moist wound healing climate, while permitting gas exchange. Wound healing assays with human skin cells confirmed a significant wound healing effect of the released insulin. In conclusion, proteohormone (e.g. insulin)-loaded fibers showed a high potential to become next generation wound dressings in the near future overcoming current challenges in wound therapy.

The present invention in particular relates to the following items.

Item 1. A method for producing core-shell fibers of biocompatible polymers comprising a bioactive proteohormone, comprising the steps of a) providing a suitable shell biocompatible polymer solution, b) providing a suitable core solution of said bioactive proteohormone, and c) coaxial electrospinning of said shell biocompatible polymer solution and said core solution of said bioactive proteohormone in order to produce said core-shell fibers.

Item 2. The method according to Item 1, wherein said bioactive proteohormone is selected from hormones involved in wound healing, human growth hormone (HGH), insulin-like growth factor-1 (IGF-1), insulin, testosterone, dehydroepiandrosterone (DHEA), and estrogen.

Item 3. The method according to Item 1 or 2, wherein said suitable core solution comprises a suitable slightly acidic buffer system, preferably comprising phosphate buffered saline (PBS), glycerol and hydrochloric acid.

Item 4. The method according to any one of Items 1 to 3, wherein said core solution further comprises suitable additional pharmaceutically active compounds, such as, for example, antimicrobial agents, such as antibacterial agents, and/or silver particles.

Item 5. The method according to any one of Items 1 to 4, wherein said biocompatible polymer is suitable for biomedical applications, in particular for uses in wound healing, based on biocompatibility, hydrophilicity, hydrophobicity, dissolution, solubility, and/or scaffold stability thereof.

Item 6. The method according to Item 5, wherein said biocompatible polymer solution comprises at least two biocompatible polymers that preferably are compatible as indicated by one singular glass transition in the first heating.

Item 7. The method according to Item 5 or 6, wherein said biocompatible polymer is selected from polymers that act as hydrogen bond donors, facilitate the interaction with water, lead to water absorption, and/or retain structural integrity.

Item 8. The method according to any one of Items 5 to 7, wherein said biocompatible polymer is selected from polycaprolactone (PCL), polyethylene oxide (PEO)/polyethylene glycol (PEG), and/or poly(D,L-lactic-co-glycolic) acid (PLGA), and biocompatible derivatives thereof.

Item 9. The method according to any one of Items 5 to 8, wherein said biocompatible polymer is dissolved in a mixture of suitable solvents, such as alcohols, such as ethanol, and chloroform.

Item 10. A coaxial core-shell fiber of biocompatible polymers comprising a bioactive proteohormone, produced according to the method according to any one of Items 1 to 9.

Item 11. The coaxial core-shell fiber according to Item 10, wherein said bioactive proteohormone is stabilized in the fiber core during storage and/or the release thereof into the wound site is prolonged.

Item 12. A patch, fibrous membrane, dressing or gauze for use with wounds, comprising coaxial core-shell fibers according to Item 10 or 11.

Item 13. The patch, fibrous membrane, dressing or gauze for use with wounds according to Item 12, exhibiting a water retention of 500 $\pm$ 25%, preferably 400 $\pm$ 25%, of the dry weight thereof.

Item 14. The patch, fibrous membrane, dressing or gauze for use with wounds according to Item 12 or 13, wherein therapeutic concentrations of said bioactive proteohormone are reached within 1 hour after application thereof, and are maintained for at least 24 hours.

Item 15. The patch, fibrous membrane, dressing or gauze for use with wounds according to any one of Items 12 to 14, wherein the surface morphology of said coaxial core-shell fibers changes after application in that small voids are formed.

Item 16. The patch, fibrous membrane, dressing or gauze for use with wounds according to any one of Items 12 to 15, wherein during use, cells form a thin layer on the very surfaces thereof, not significantly invading into the patch, fibrous membrane, gauze for use with wounds or wound dressing, and exhibiting a healthy morphology.

Item 17. The patch, fibrous membrane, dressing or gauze for use with wounds according to any one of Items 12 to 16, wherein said patch, fibrous membrane, dressing or gauze allows for a regular change of the dressing without fusion of wound and dressing, and without inflicting tissue damage when being removed.

Item 18. The patch, fibrous membrane, dressing or gauze for use with wounds according to any one of Items 12 to 17, wherein said wound is a chronic wound, for example a diabetic ulcer.

Item 19. Use of coaxial core-shell fibers according to Item 10 or 11 in wound therapy systems.

Item 20. A bioactive proteohormone for use in the therapy of wounds, comprising administering said proteohormone using the patch, fibrous membrane, dressing or gauze according to any one of Items 12 to 18.

Item 21. The bioactive proteohormone for use according to Item 20, wherein said proteohormone is selected from hormones involved in wound healing, human growth hormone (HGH), insulin-like growth factor-1 (IGF-1), insulin, testosterone, dehydroepiandrosterone (DHEA), and estrogen.

Item 22. The bioactive proteohormone for use according to Item 20 or 21, wherein said wound is a chronic wound, for example a diabetic ulcer.

Item 23. A method of treating or preventing a wound or promoting wound healing in a mammalian patient, such as a human, comprising administering a proteohormone promoting wound healing using the patch, fibrous membrane, dressing or gauze according to any one of Items 12 to 18 to said patient in need thereof.

Item 24. The method according to Item 23, wherein said proteohormone is selected from hormones involved in wound healing, human growth hormone (HGH), insulin-like growth factor-1 (IGF-1), insulin, testosterone, dehydroepiandrosterone (DHEA), and estrogen and combinations thereof.

Item 25. The method according to Item 23 or 24, wherein said treatment or prevention is in combination with suitable additional pharmaceutically active compounds, such as, for example, antimicrobial agents, such as antibacterial agents, and/or silver particles.

Item 26. The method according to any one of Items 23 to 25, wherein said wound is a chronic wound, for example a diabetic ulcer.

The present invention will now be described further in the following examples with reference to the accompanying Figures, nevertheless, without being limited thereto. For the purposes of the present invention, all references as cited herein are incorporated by reference in their entireties.

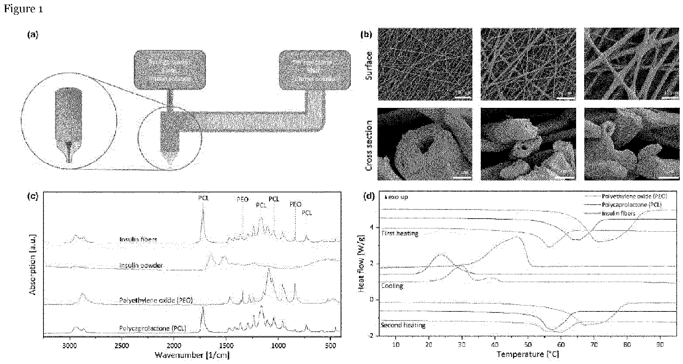

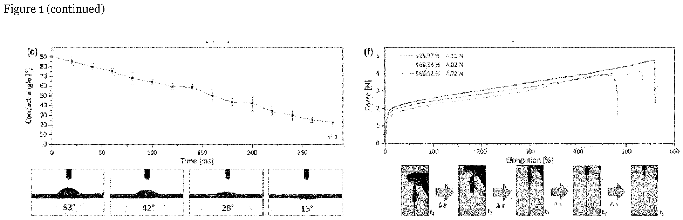

Figure 1 shows: (a) Scheme of the coaxial electrospinning setup (b) SEM images of the fiber scaffold surface, displaying randomly aligned fibers with homogeneous fiber diameter distribution (top) and the core-shell structure of the fibers in cross sections (bottom). (c) ATR-IR spectra of insulin fibers, insulin powder and the two polymers used in the shell solution (PEO and PCL). (d) Thermograms of insulin fibers and the two polymers used (PEO and PCL). The first heating only shows one glass transition for the fibers, confirming the compatibility of the two polymers. Second heating displays both polymers, after they have crystallized next to each other during the cooling section. (e) Contact angle of water dispensed onto the fiber scaffolds. The water is rapidly absorbed into the scaffold within approx. 300 ms, visualized by representative images below. (f) Tensile force diagram, displaying elongation of the fiber mats and the applied force, with representative images below the graph. A phase of elastic deformation with fiber reorientation is followed by extensive plastic deformation before the force suddenly drops, indicating rupture ($t_5$). Maximum elongation and force at break are displayed.

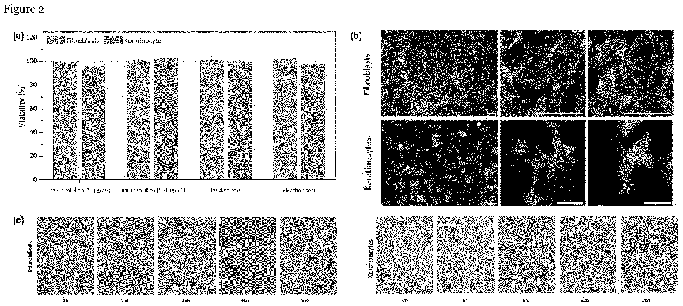

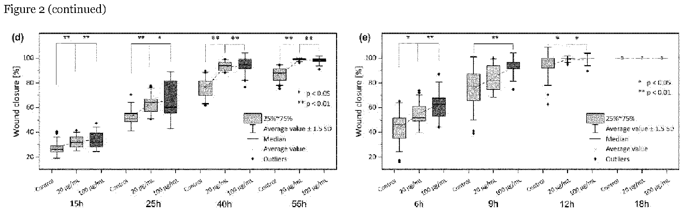

Figure 2 shows: (a) Cell viability assay with human fibroblasts and keratinocytes confirming unobstructed cell viability for cells cultivated with insulin or directly seeded onto insulin fibers and unloaded fibers (b) Fluorescence microscopy images depicting the cells seeded on insulin fibers. Actin staining is displayed in green and nuclei staining in blue, respectively. Scale bars are 150 $\mu$m. (c) Scratch assay images of primary human fibroblasts and keratinocytes at different time points during defect area closure at 10x resolution. Fibroblasts branched out into the defect area to close the gap, where keratinocytes strived to close the gap by merging rather linear borders. (d) Relative wound closure of fibroblasts over time in relation to initial wound area. Concentrations of 20 and 100 $\mu$g/mL insulin significantly accelerated wound healing, compared to the control experiments. While cells treated with insulin achieved full closure, the control group only achieved approx. 86% closure within 55 hours. (e) Relative wound closure of keratinocytes cells over time in relation to initial wound area. Concentrations of 20 and 100 $\mu$g/mL showed significantly accelerated defect area closure compared to control. Complete confluence was recovered after at least 18 hours for all experiments.

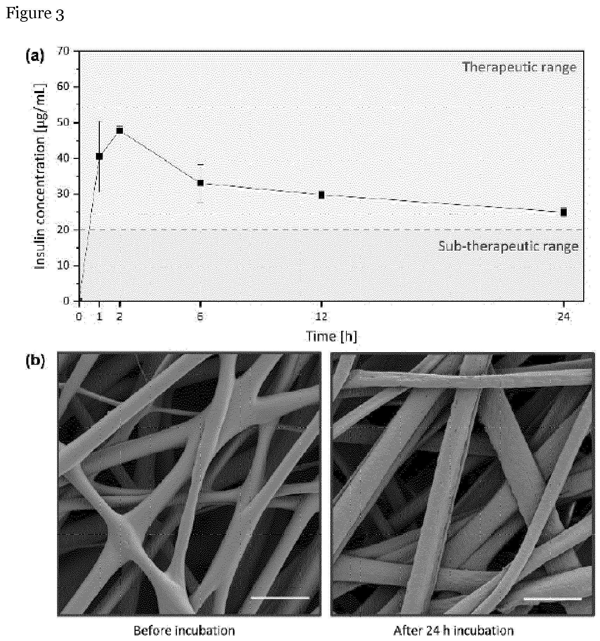

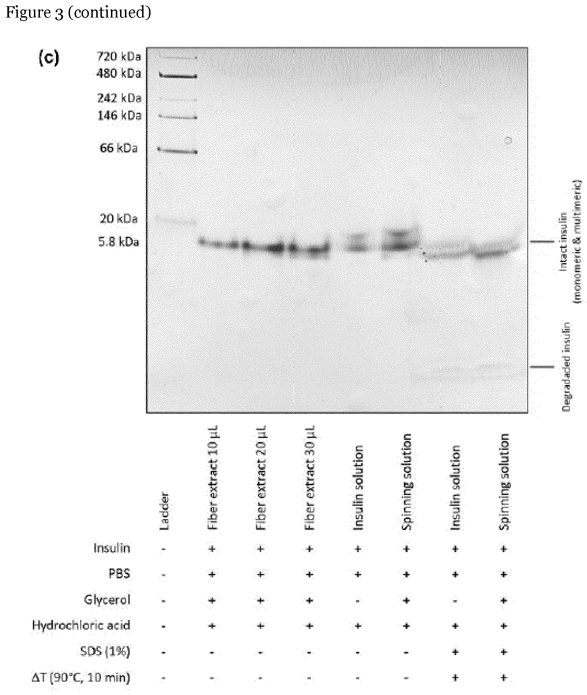

Figure 3 shows: (a) Insulin release kinetics. Therapeutic insulin concentrations were detected within the first hour and maintained for 24 h, matching common wound dressing change intervals. (b) SEM images of insulin fibers before and after incubation in PBS for 24 hours at 32°C. The surface of the incubated fibers is quite smooth, and small voids have formed, most likely due to the dissolution of the water soluble polymer PEO, thus facilitating the release of insulin. Scale bars are 10 $\mu$m (c) Electrophoresis experiments confirm that insulin is released from the fibers in its intact form, as the bands appear at the same height as the positive controls. As negative controls insulin was denatured using SDS and heat. To some extent, negative control bands also appear at the same height as the positive control, as peptide refolding takes place during the native electrophoresis conditions. The degradation product of the negative controls is visible at the bottom of the gel. Double bands are most likely attributed to dimer and oligomer formation.

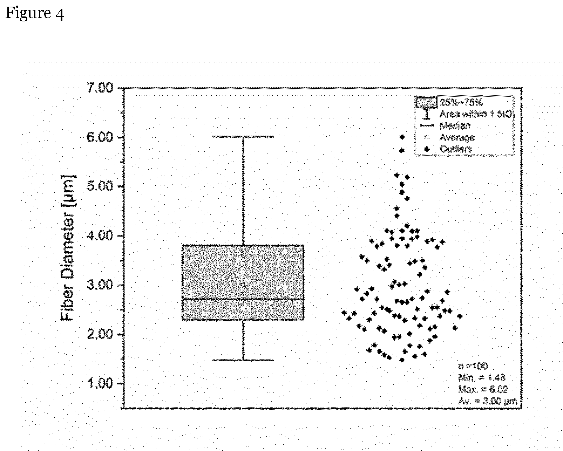

Figure 4 shows the fiber diameter distribution of the samples as produced.

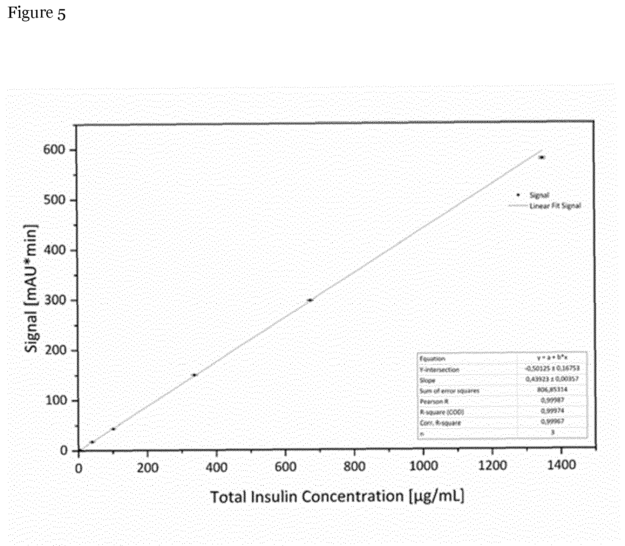

Figure 5 shows the HPLC calibration curve for insulin as detected.

**Examples**

**Materials and Methods**

The following materials were used for this invention. Ethanol (#17C104016), methanol HiPerSolv CHROMANORM (#18H074016) and chloroform (#18E044018) were purchased from VWR (Darmstadt, Germany). Polyethylene oxide 600 kDa (#MKBR0031V), polycaprolactone 80 kDa (#MKBR4731V), insulin powder human recombinant (#19D227), fetal calf serum (#BCBX5313), Dulbecco's phosphate buffered saline (#RNBH7500), cytotoxicity detection kit (LDH, Roche, #40490800), Alexa Fluor 568 phalloidin (Invitrogen, #1987203), 4',6-diamidino-2-phenylindole (Invitrogen, #2031179) and native PAGE gel (Invitrogen, Novex WedgeWell 16% tris-glycine, #20032442), tris-glycine native running buffer (Novex, #2166766), acetonitrile chromasolv gradient (Honeywell, #J1580), native tris-glycine sample buffer (Novex, #2152692) and Triton X-100 (#SLBW7103) were obtained from Sigma Aldrich (Steinheim, Germany). Sterican® blunt-tip needles, 22G were purchased from B. Braun Melsungen (Melsungen, Germany). Hydrochloric acid $1_M$ (#HC73956057), anhydrous glycerol (#K52293328011) and $KH_2PO_4$ (#AM1089577736) were purchased from Merck KGaA (Darmstadt, Germany). Dulbecco's modified Eagle medium (DMEM) (#2045120) was purchased from Gibco® Life Technologies (Darmstadt, Germany). Formaldehyde methanol-free 30% (#428270168) was obtained from Carl Roth GmbH (Karlsruhe, Germany).

Primary human dermal fibroblasts (NHDF, cat. no. C-12302) were purchased from PromoCell GmbH (Heidelberg, Germany). The spontaneously immortalized human keratinocyte cell line HaCaT (human, adult, low calcium, high temperature keratinocytes) was kindly donated by Prof. N. Fusenig (German Cancer Research Center, Heidelberg, Germany)[1]. CELLSTAR® cell culture flasks T75 658175 (#E1911344) and 96-well flat bottom plates (#E130304K) were purchased from Greiner Bio-One (Frickenhausen, Germany). 96-well ImageLock microplates for the scratch assay (18021401) were purchased from Essen BioScience (Essen, Germany).

**Coaxial Electrospinning**

For the preparation of the shell solution, polyethylene oxide (PEO, 600 kDa, 1.8% w/v) and polycaprolactone (PCL, 80 kDa, 10.2% w/v) were dissolved in ethanol and chloroform (1:1 v/v). After overnight dissolution, the solution was vortexed until a homogeneous, slightly opaque polymer solution had formed. The solution was briefly degassed in an ultrasonic bath and filled into a Hamilton glass syringe (10 mL), connected via polytetrafluoroethylene (PTFE) Luer lock adapters and PTFE tubing with the shell port of a stainless steel coaxial spinning needle. For the preparation of the core solution, 15 mg powdered insulin (human, recombinant) were dissolved (10 mg/mL) in 750 $\mu$L PBS, acidified with 25 $\mu$L HCl and mixed with 530 $\mu$L glycerol (85%) under sterile conditions to obtain a total insulin concentration of 11.5 mg/mL. The solution was filled into a Hamilton glass syringe (1 mL), which was then connected to the core port of the concentric spinning needle. Solutions were electrospun at 7 kV with flow rates of 0.28 mL/h (core) and 2.8 mL/h (shell) on a rotating metal drum collector (250 RPM) for 2 hours. The fibers were removed from the collector, wrapped in aluminum foil and stored in a desiccator at 4°C until further use.

For comparative studies, unloaded fibers were electrospun, using the shell solution of the coaxial insulin fibers. Unloaded fibers were spun at the same spinning conditions described above, but with a uni-axial 22G single use, blunt tip needle.

**Scanning Electron Microscopy and Fiber Diameter**

Scanning electron microscopy images were acquired on a field emission scanning electron microscope (S 4500, Hitachi, Chiyoda, Japan) at 5 kV. Samples were gold sputtered before visualization. Fiber diameters were determined using Fiji (ImageJ) (see figure S2.3)

## Attenuated Total Reflection Infrared (ATR-IR) Spectroscopy

ATR-IR spectra were recorded at multiple spots across the fiber mat with an ALPHA II Platinum FT-IR spectrometer (Bruker, Billerica, MA, USA) utilizing OPUS-TOUCH software. For each data point, twenty spectra were recorded and averaged.

## Differential Scanning Calorimetry (DSC)

Thermograms were recorded with a DSC 3 (Mettler Toledo, Columbus, OH, USA), equipped with an intracooler TC100. Samples of approx. 15 mg were weighed into 40 $\mu$L aluminum pans before punch perforating the lid and cold-welding. Samples were heated from 0-100°C with a heating rate of 10°C/min and cooled from 100-0°C with a cooling rate of 10°C/min, before being heated again from 0-100°C with a heating rate of 10°C/min. Data evaluation was performed with the software STAR$^e$ (Mettler Toledo, Columbus, OH, USA).

## Contact Angle

Contact angle measurements were performed with a dynamic contact angle measurement device (OCA 15LJ, Dataphysics, Filderstadt, Germany). Samples were clamed in a designated foil and paper mount. A straight tip needle (outer diameter 31 mm) was used to dispense 10 $\mu$L Milli-Q water drops. The drops were then transferred to the fiber mat surface and high-speed video recording and analysis was used for dynamic contact angle determination until water absorption was completed and contact angles could no longer be determined. Experiments were performed in threefold execution.

## Analysis of Thickness and Mechanical Properties

Scaffold thickness was measured with a thickness measurement device (MiniTest 735 / Sensor F5, Elektro-Physik, Cologne, Germany). For thickness measurement, the fiber mats were placed on a metal plate and covered with a thickness-tarred glass cover slip. Each specimen was measured at different spots for thickness in eightfold determination.

Analysis of the mechanical fiber properties was performed on a vertical single column tensile tester (5942, Instron, Norwood, MA, USA). Fibers were clamped with rubber jaw faces installed in pneumatic grips. A 50 N load cell was used for the experiments. Experiments were carried out in threefold determination. Specimens of 40 mm x 10 mm x 86.00 $\pm$ 14.56 $\mu$m (H x W x D) were used for the experiments. Specimens were pre-stretched to 50 mN with 2 mm/min before resetting travel and force. Specimens were stretched with 10 mm/min until break (force drop to 40%). Travel and force were recorded simultaneously.

## Cell Culture

Human dermal fibroblasts (NHDF) and human keratinocytes (HaCaT) were routinely cultured in high-glucose Dulbecco's Modified Eagle Medium (DMEM) basal medium supplemented with 10% fetal calf serum (FCS). The cells were maintained at 37°C in a humidified atmosphere containing 5% $CO_2$. Medium was refreshed every two to three days.

For cell viability testing and wound healing assays, the cells were maintained under serum-deprived conditions, as nutrition starvation is typically observed upon tissue injury due to disruption of the local blood supply. Hence, the FCS proportion of the culture medium was weekly gradually reduced from initial 10% (v/v) to 2.5% (v/v) for fibroblasts as final deprivation state and 1% (v/v) for keratinocytes, respectively. Fibroblasts could not be deprived down to 1% (v/v) FCS supplementation, because cell growth arrest was already apparent followed by rapid induction of cell death.

For cell-fiber interactions studies, autoclaved silicone rings were used to hold down 12 mm fiber mat punches, which were previously UV-sterilized, to the bottom of a 24-well plate. The rings were then filled with a cell suspension in DMEM with 10% FCS. For keratinocytes, 35000 cells/500 $\mu$L were used and for fibroblasts 25000 cells/500 $\mu$L were used.

## Cell Viability

For cell viability testing after incubation with insulin, the FCS-deprived cells were seeded in 96-well plates with 35,000 cells in 100 $\mu$L medium/well for keratinocytes and 25,000 cells in 100 $\mu$L medium/well for fibroblasts. A confluent monolayer was obtained after 24h of incubation. Cell culture medium was aspirated and wells were filled with 100 $\mu$L

FCS-free DMEM, supplemented with 20 μg/mL or 100 μg/mL insulin, respectively.

After 23 hours, supernatants of the positive control were aspirated and 100 μL FCS-reduced DMEM, supplemented with 1% Triton-X, was pipetted into the wells. After 1 hour of further incubation, all supernatants were collected and centrifuged for 10 minutes at 250 G.

Cytotoxicity testing was performed with a cytotoxicity detection kit according to the manufacturer's instructions. Absorption was measured at 492 nm with kinetic measurements in a plate reader (Spark, TECAN, Männedorf, Switzerland). The last measurement in the linear range was used for further calculations.

For cell-fiber interaction studies, cells were seeded on fibers (see section S2.8 for further information) and incubated at 37°C and 5% $CO_2$ for 72 hours.

Positive and negative controls were performed using just silicone rings and cell suspension.

After 71 hours, supernatants of the positive controls were aspirated and 500 μL DMEM with 10% FCS, supplemented with 1% Triton-X, was pipetted into the rings. The further processing was comparable as already described for the pure compound cytotoxicity testing based on cell monolayers.

Cell viability was calculated with equation (1):

$$\text{Equation (1)} \qquad Viability\ [\%] = 1 - \frac{exp.value - low\ control}{high\ control - low\ control}\ x\ 100\%$$

**Fluorescence Microscopy**

To evaluate the interaction of the cells with the fabricated fibers, cell behavior was analyzed based on visualization of cellular compartments focusing on the cytoskeleton and cell nuclei. Briefly, fibers separately co-cultured with skin cells (fibroblasts and keratinocytes, respectively) were washed twice with sterile PBS and fixed with 3.7% formaldehyde in PBS for 15 minutes. All incubation steps were performed at room temperature. After repeated rinsing with PBS, the samples were either stored at this stage in PBS at 4°C until further use or the further staining processing was considered. Prior to the fluorescence dye incubation, cell permeabilization with 0.1% Triton-X for 10 minutes was performed followed by repeated washing steps with PBS. Alexa Fluor 568 Phalloidin solution (1:40 dilution in PBS) was selected for cell cytoskeleton labelling (60 minutes incubation). After repeated washing with PBS, cell nuclei were counterstained with DAPI (1:1000 dilution in PBS) for 60 minutes and rinsed again with PBS. Samples were visualized with a DMi8 fluorescence microscope (Leica Microsystems, Wetzlar, Germany).

Table 1: Preparation of native PAGE samples

| Well | 1 | 2 | 3 | 4 | 5 | 6 | 7 | 8 | 9 | 10 |
|---|---|---|---|---|---|---|---|---|---|---|
| Designation | Blank | Ladder | Extract, low load | Extract, medium load | Extract, high load | InsPow PBS, 1:9 diluted | Spinning solution, 1:9 diluted | InsPow PBS, 1:9 diluted, 1% SDS | Spinning solution, 1:9 diluted, 1% SDS | Blank |
| Temperature | - | - | - | - | - | - | - | 10 min @ 90°C | 10 min @ 90°C | - |
| Sample | - | 1.2 μL | 10 μL | 20 μL | 30 μL | 2 μL | 2 μL | 2 μL | 2 μL | - |
| Water | - | 19 μL | 10 μL | - | - | 18 μL | 18 μL | 28 μL | 28 μL | - |
| Loading Dye | - | 20 μL | 20 μL | 20 μL | 30 μL | 20 μL | 20 μL | 25 μL | 25 μL | - |
| SDS (10%) | - | - | - | - | - | - | - | 5 μL | 5 μL | - |

**Results**

**Wound Healing**

For efficacy testing of the developed insulin-loaded electrospun fibers, in vitro scratch wound assays were performed with keratinocytes and fibroblasts as the two predominant cell types in skin tissue and key players for the wound healing process. The experiments were separately performed with FCS-deprived keratinocytes (1%) and fibroblasts (2.5%), respectively. Cells were seeded in 96-well plates with 35000 cells in 100 $\mu$L medium/well for keratinocytes and 25000 cells in 100 $\mu$L medium/well for fibroblasts to reach a confluent monolayer within 24h of culture. Experiments were carried out with passage number 78, 80 and 82 for keratinocytes and 8, 11 and 12 for fibroblasts, respectively.

Prior to experimental scratch wounding, the cells were preincubated with PBS for 5 minutes to facilitate calcium mediated cell detachment from the well bottom. Subsequently, a cell-free defect area representing the "wound" was prepared by disrupting the cell monolayer in a straight line using a commercially available scratch tool (WoundMaker™, Essen BioScience, Essen, Germany). To remove detached cells and scratch debris, the samples were washed twice with sterile PBS. Potential wound healing promoting effects of insulin were evaluated using FCS-free DMEM supplemented with 20 $\mu$g/mL or 100 $\mu$g/mL insulin. Cells only treated with basal medium (DMEM, with 1% FCS for HaCaT or 2.5% FCS for NHDF, respectively) served as negative control.

Wells were imaged in an IncuCyte S3 (Essen BioScience, Essen, Germany)) with a 10x lens every hour for 24 hours (HaCaT) and 55 hours (for NHDF), respectively.

**High Pressure Liquid Chromatography (HPLC) Analysis**

HPLC analysis was performed on an Dionex UltiMate 3000 (ThermoFisher, Dreieich, Germany) with a temperature-controlled autosampler, using a 100 mm RP-18 column (BDS Hypersil C18, 100 x 4.6 mm, 3 $\mu$m particle size, ThermoFisher Scientific, Dreieich, Germany). Samples were incubated at 32°C and an injection volume of 20 $\mu$L was used. The mobile phase (62% 0.1M $KH_2PO_4$ in Milli-Q, 26% acetonitrile and 12% methanol) was pumped with 1 mL/min for a total runtime of 10 minutes. Absorption was measured at 214 nm, using a diode array detector.

A calibration was performed by dissolving 10 mg insulin powder (human recombinant) in 1 mL PBS with 15 $\mu$L 1M HCl to enable dissolution. This stock solution was diluted with PBS to concentrations of 4.725, 40.5, 101.5, 337.5, 675 and 1350 $\mu$g/mL. Coefficient of correlation was determined to be 0.99987, thus sufficient for quantification (see figure S2.12)

For release testing, fiber mats were cut into 29 x 21 mm rectangular pieces. After weighing, these cuts were rolled and transferred into brown glass HPLC vials. As soon as the vials were filled with 1.5 mL sterile PBS buffer, the fiber mats expanded, closely laying on the sides of the HPLC vials. The vials were then placed in the HPLC autosampler at 32°C, mimicking the human skin surface temperature. Samples of 20 $\mu$L were taken after 1h, 2h, 6h, 12h and 24h in threefold determination. Three independent experiments were carried out. Curves were integrated manually.

**Gel Electrophoresis**

Insulin was extracted from the fibers by placing 200 mg of fiber mat in 1 mL PBS at room temperature. Reference samples were prepared by preparing the core spinning solution according to the above section (spinning solution) or by the same method, but replacing the glycol with PBS (InsPow PBS). Both reference samples were diluted 1:9 before preparing the loading solutions. The loading solutions were prepared according to table 1, and transferred into the gel wells.

The gel was run at 225V and 300 mA for 50 minutes before staining with a Coomassie blue staining solution for 25 minutes, before destaining several times with a destaining solution (10% acetic acid and 10% ethanol in deionized water) and imaging with a benchtop office scanner.

**Statistical Analysis**

All results presented as their mean $\pm$ standard deviation (SD). Statistically significant differences between experimental groups were analyzed using the Wilcoxon signed-rank test, which requires no normality of the data and is specifically applied for small data sets. P-values < 0.05 were considered to be statistically significant and indicated by (*, p < 0.01 **).

All calculations were performed using Microsoft Office Excel 2016 and Origin Pro 2019.

The present inventors designed insulin-loaded core-shell fibers as novel wound dressings. Two biocompatible polymers, namely the rather hydrophobic polycaprolactone (PCL, 80 kDa, 10.8% w/v) and hydrophilic polyethylene oxide (PEO, 600 kDa, 1.2% w/v) were dissolved in a mixture of ethanol and chloroform (1:1 v/v) and used as the shell solution.

The core solution was prepared by dissolving human recombinant insulin in phosphate buffered saline (PBS), glycerol and hydrochloric acid. With a coaxial setup (Figure 1a), the inventors physically separated the aqueous core (insulin solution) from the organic solvent in the shell polymer solution. This does not only protect the insulin during processing, but also introduces an insulin-free shell layer, leading to stabilization of insulin in the fiber core during storage and prolonging its release into the wound site.[16] The physico-chemical properties of these polymer fibers were optimized for biomedical application in terms of biocompatibility, hydrophilicity and scaffold stability.

Scanning electron microscopy (SEM) revealed that the fibers are randomly aligned, feature a smooth surface and a narrow fiber diameter distribution of $3 \pm 1$ $\mu$m (Figure 5). The fiber intersections are clearly merged, improving the mechanical strength of the fiber mats (Figure 1b, surface visualization). In order to resolve the core-shell structure of the fibers, the scaffolds were frozen in liquid nitrogen to reduce ductility, then cryo-cut and visualized by SEM. Cross-sections of fibers show the hollow core, from which the water within the aqueous core solution evaporated during dry storage. (Figure 1b, cross section).

Attenuated total reflection infrared (ATR-IR) spectroscopy proofed that both polymers are clearly present in the spectrum of the fibers and the spectral composition remains consistent throughout the fiber scaffold (Figure 1c).

Thermograms acquired by differential scanning calorimetry (DSC) depict that the two polymers are compatible, visualized by one singular glass transition peak during first heating. In the course of cooling, the two liquid polymers crystallize apart from each other and show overlapping, yet clearly separable glass transitions during second heating (Figure Id).

In order to characterize the interaction with wound exudate, contact angle measurements were performed. Although the majority of the polymer components is not water soluble, both polymers act as hydrogen bond donors, facilitating the interaction with water and leading to rapid water absorption by the scaffold, while retaining the structural integrity of the fibers (Figure 1e). Water absorption is followed by extensive water retention of $400 \pm 25\%$ of the dry dressing weight. This is crucial on the one hand for the uptake of wound exudate and establishment of a moist wound environment, and on the other hand to prevent the dressing from sticking to the wound, thus paving the way for painless and non-destructive dressing changes.

When applying wound dressings to body areas with a high degree of movement like joints, it is vital that the system is flexible enough to follow the physiological movement of the patient, while keeping the dressing in close contact with the wound and reducing friction. To investigate the mechanical properties of these novel fibers, ductility was quantified by testing the tensile strength and elasticity, while stretching the scaffolds until breakage, simultaneously measuring elongation and force. When stretched, the individual fibers undergo reorientation in force direction (elastic deformation), before entering a stage of extensive plastic deformation. The scaffolds exhibit a sharp breakpoint at maximum elongation of $517 \pm 36$ % and maximum force of $4.29 \pm 0.31$ N (Figure If).

Further, the biological compatibility and performance of the fibrous drug delivery system was evaluated in cell experiments with human keratinocytes and fibroblasts, the two prevalent cell types forming the human skin. In order to confirm that neither insulin nor the fibers reduce cell viability, cells were either incubated with cell culture medium containing insulin in different concentrations or were directly seeded onto previously sterilized electrospun fiber mats as above. Subsequent analysis of cytotoxicity markers did not indicate any significant reduction in cell viability for keratinocytes or fibroblasts (Figure 2a).[17]

Afterwards, scaffolds were fixed and stained to visualize actin skeleton and cell nuclei, respectively, as above. Fluorescence microscopy images revealed that cells form a thin layer on the very surface of the fiber mat, not significantly invading into the mats and exhibiting healthy morphology (Figure 2b). For the future clinical application, this is ideal, as cells accept the dressing as a guidance to close the wound, but allow for regular change of the dressing without fusion of wound and dressing, inflicting tissue damage when being removed.

As a next step, in vitro wound healing studies were performed with human skin cells. Such scratch assays exhibit a well-established surrogate for the prediction of wound healing effects in the human body.[18,19] Briefly, scratches in confluent monolayers of keratinocytes and fibroblasts were created, resulting in a defect (wound) area. The cell monolayers were then treated with cell culture medium containing insulin in concentrations of 20 $\mu$g/mL and 100 $\mu$g/mL, respectively. The defect area closure was monitored with time-lapse video analysis (Figure 2c).

Ahead of the experiments, cells were gradually deprived of fetal calf serum (FCS), to exclude secondary effects of growth factors in the cell culture medium, thus isolating the effect of therapeutically supplemented insulin on wound healing and mimic the pathologic lack of growth factors in the wound site.

The wound healing performance was quantified by determining the relative remaining wound area at different time points. For fibroblasts, significantly accelerated healing was observed and wounds treated with insulin reached complete closure after 55 hours, compared to $86.29 \pm 5.39$ % closure in the control group (Figure 2d).

Keratinocytes showed significantly improved wound closure with $92.80 \pm 7.18\%$ after 9 hours for 100 $\mu$g/mL, compared to $75.59 \pm 16.50\%$ for the control group, achieving complete closure after 12 hours, with 100 $\mu$g/mL showing a greater effect than 20 $\mu$g/mL (Figure 2e).

Even though scratch assays are well established as predictors for wound healing in the human body, the wound area for such in vitro tests is generally small. Therefore, an even more pronounced effect can be expected in the human body with longer treatment periods. It could thus be demonstrated that insulin leads to improved wound closure for the applied therapeutic range of insulin (20 - 100 μg/mL) and neither insulin nor the fibers exhibit a cytotoxic potential.

In order to investigate the release kinetics of insulin from the fibers, fibers were placed in PBS and high performance liquid chromatography (HPLC) analyses of the release testing medium were performed with a method described previously.[20] Within 1 hour of incubation, therapeutic insulin concentrations were detected in the release medium and maintained for 24 hours, providing an ideal therapeutic window with respect to clinical wound dressing change intervals of 24 hours (Figure 3a). Comparing SEM images of the fiber surface before and after release testing, surface morphology of the fibers has changed. Small voids have formed, most likely due to the dissolution of PEO, thus paving the way for the release of insulin from the core through the shell layer (Figure 3b).

Native polyacrylamide gel electrophoresis (PAGE) experiments were performed to proof integrity (and thus therapeutic activity) of the released insulin. The experiment was carried out with the medium after insulin release from the fiber mats in different concentrations, freshly prepared insulin solution and the core spinning formulation used (positive controls), as well as solutions with insulin intentionally denaturized at 80°C by addition of sodium dodecyl sulfate (negative control). The results indicate that insulin is released from the fibers in its intact form, as the bands of the insulin in release medium appear at the same height as the positive controls. Although the negative control was denaturized, proteins are known to fold back into their native conformation when being re-introduced to a native environment, as present during the gel running experiment, which is assumed to be the reason why the majority of the protein is detected at the same height as the positive control (Figure 3c). As previously shown, insulin shows a pH and concentration dependent self-assembly into dimers and oligomers, which could be held accountable for the exhibition of two discrete bands for the freshly prepared insulin solution.[21]

**References as cited**

[1] J. Posnett, F. Gottrup, H. Lundgren, G. Saal, Journal of Wound Care 2009, 18, 154.

[2] S. Schreml, R.-M. Szeimies, L. Prantl, M. Landthaler, P. Babilas, Journal of the American Academy of Dermatology 2010, 63, 866.

[3] S. R. Nussbaum, M. J. Carter, C. E. Fife, J. DaVanzo, R. Haught, M. Nusgart, D. Cartwright, Value in Health 2018, 21, 27.

[4] B. A. Mast, G. S. Schultz, Wound Rep. Reg. 1996, 4, 411.

[5] S. Das, G. Singh, M. Majid, M. B. Sherman, S. Mukhopadhyay, C. S. Wright, P. E. Martin, A. K. Dunn, A. B. Baker, Adv. Healthcare Mater. 2016, 5, 2248.

[6] M. Hrynyk, R. J. Neufeld, Burns 2014, 40, 1433.

[7] A. Oliveira, S. Simões, A. Ascenso, C. P. Reis, Journal of Dermatological Treatment 2020, 1.

[8] S. Barrientos, H. Brem, O. Stojadinovic, M. Tomic-Canic, Wound Repair Regen 2014, 22, 569.

[9] B. D. Almquist, S. A. Castleberry, J. B. Sun, A. Y. Lu, P. T. Hammond, Adv. Healthcare Mater. 2015, 4, 2090.

[10] G. Gainza, S. Villullas, J. L. Pedraz, R. M. Hernandez, M. Igartua, Nanomedicine: Nanotechnology, Biology and Medicine 2015, 11, 1551.

[11] G. Andreas, W. J. H, Angewandte Chemie International Edition 2007, 46, 5670.

[12] M. Liu, X.-P. Duan, Y.-M. Li, D.-P. Yang, Y.-Z. Long, Materials Science and Engineering: C 2017, 76, 1413.

[13] F. Croisier, G. Atanasova, Y. Poumay, C. Jérôme, Adv. Healthcare Mater. 2014, 3, 2032.

[14] T. K. Hunt, H. W. Hopf, Surgical Clinics of North America 1997, 77, 587.

[15] S. Dhivya, V. V. Padma, E. Santhini, BioMed 2015, 5, 22.

[16] D. Han, A. J. Steckl, ChemPlusChem 2019, 84, 1453.

[17] T. Decker, M.-L. Lohmann-Matthes, Journal of Immunological Methods 1988, 115, 61.

[18] C.-C. Liang, A. Y. Park, J.-L. Guan, Nat Protoc 2007, 2, 329.

[19] J. E. N. Jonkman, J. A. Cathcart, F. Xu, M. E. Bartolini, J. E. Amon, K. M. Stevens, P. Colarusso, Cell Adhesion & Migration 2014, 8, 440.

[20] A. Najjar, M. Alawi, N. AbuHeshmeh, A. Sallam, Advances in Pharmaceutics 2014, 2014, 1.

[21] A. K. Attri, C. Fernández, A. P. Minton, Biophysical Chemistry 2010, 148, 28.

**Claims**

1. A method for producing core-shell fibers of biocompatible polymers comprising a bioactive proteohormone, comprising the steps of

a) providing a suitable shell biocompatible polymer solution,

b) providing a suitable core solution of said bioactive proteohormone, and

c) coaxial electrospinning of said shell biocompatible polymer solution and said core solution of said bioactive proteohormone in order to produce said core-shell fibers.

2. The method according to claim 1, wherein said bioactive proteohormone is selected from hormones involved in wound healing, human growth hormone (HGH), insulin-like growth factor-1 (IGF-1), insulin, testosterone, dehydroepiandrosterone (DHEA), and estrogen.

3. The method according to claim 1 or 2, wherein said suitable core solution comprises a suitable slightly acidic buffer system, preferably comprising phosphate buffered saline (PBS), glycerol and hydrochloric acid.

4. The method according to any one of claims 1 to 3, wherein said core solution further comprises suitable additional pharmaceutically active compounds, such as, for example, antimicrobial agents, such as antibacterial agents, and/or silver particles.

5. The method according to any one of claims 1 to 4, wherein said biocompatible polymer is suitable for biomedical applications, in particular for uses in wound healing, based on biocompatibility, hydrophilicity, hydrophobicity, dissolution, solubility, and/or scaffold stability thereof, and wherein preferably said biocompatible polymer solution comprises at least two biocompatible polymers that preferably are compatible as indicated by one singular glass transition in the first heating.

6. The method according to claim 5, wherein said biocompatible polymer is selected from polymers that act as hydrogen bond donors, facilitate the interaction with water, lead to water absorption, and/or retain structural integrity, such as, for example, selected from polycaprolactone (PCL), polyethylene oxide (PEO)/polyethylene glycol (PEG), and/or poly(D,L-lactic-co-glycolic) acid (PLGA), and biocompatible derivatives thereof.

7. The method according to any one of claims 5 or 6, wherein said biocompatible polymer is dissolved in a mixture of suitable solvents, such as alcohols, such as ethanol, and chloroform.

8. A coaxial core-shell fiber of biocompatible polymers comprising a bioactive proteohormone, produced according to the method according to any one of claims 1 to 7, wherein preferably said bioactive proteohormone is stabilized in the fiber core during storage and/or the release thereof into the wound site is prolonged.

9. A patch, fibrous membrane, dressing or gauze for use with wounds, comprising coaxial core-shell fibers according to claim 8, wherein preferably therapeutic concentrations of said bioactive proteohormone are reached within 1 hour after application thereof, and are maintained for at least 24 hours.

10. The patch, fibrous membrane, dressing or gauze for use with wounds according to claim 9, wherein the surface morphology of said coaxial core-shell fibers changes after application in that small voids are formed.

11. The patch, fibrous membrane, dressing or gauze for use with wounds according to claim 9 or 10, wherein during use cells form a thin layer on the very surfaces thereof, not significantly invading into the patch, fibrous membrane, gauze for use with wounds or wound dressing, and exhibiting a healthy morphology.

12. The patch, fibrous membrane, dressing or gauze for use with wounds according to any one of claims 9 to 11, wherein said patch, fibrous membrane, dressing or gauze allows for a regular change of the dressing without fusion of wound and dressing, and without inflicting tissue damage when being removed.

13. The patch, fibrous membrane, dressing or gauze for use with wounds according to any one of claims 9 to 12, wherein said wound is a chronic wound, for example a diabetic ulcer.

14. Use of coaxial core-shell fibers according to claim 10 or 11 in wound therapy systems.

15. A bioactive proteohormone for use in the therapy of wounds, comprising administering said proteohormone using the patch, fibrous membrane, dressing or gauze according to any one of claims 9 to 13, wherein preferably said proteohormone is selected from hormones involved in wound healing, human growth hormone (HGH), insulin-like growth factor-1 (IGF-1), insulin, testosterone, dehydroepiandrosterone (DHEA), and estrogen, and wherein preferably said wound is a chronic wound, for example a diabetic ulcer.

Figure 1

Figure 1 (continued)

Figure 2

Figure 2 (continued)

Figure 3

(a)

(b)

Before incubation

After 24 h incubation

Figure 3 (continued)

**(c)**

Ladder labels: 720 kDa, 480 kDa, 242 kDa, 146 kDa, 66 kDa, 20 kDa, 5.8 kDa

Right side labels: Intact insulin (monomeric & multimeric); Degraded insulin

| | Ladder | Fiber extract 10 µL | Fiber extract 20 µL | Fiber extract 30 µL | Insulin solution | Spinning solution | Insulin solution | Spinning solution |
|---|---|---|---|---|---|---|---|---|
| Insulin | - | + | + | + | + | + | + | + |
| PBS | - | + | + | + | + | + | + | + |
| Glycerol | - | + | + | + | - | + | - | + |
| Hydrochloric acid | - | + | + | + | + | + | + | + |
| SDS (1%) | - | - | - | - | - | - | + | + |
| ΔT (90°C, 10 min) | - | - | - | - | - | - | + | + |

Figure 4

Figure 5

Europäisches
Patentamt

European
Patent Office

Office européen
des brevets

# EUROPEAN SEARCH REPORT

Application Number

EP 20 20 0785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YIN LIHUA ET AL: "Physicochemical and biological characteristics of BMP-2/IGF-1-loaded three-dimensional coaxial electrospun fibrous membranes for bone defect repair", JOURNAL OF MATERIALS SCIENCE: MATERIALS IN MEDICINE, SPRINGER NEW YORK LLC, UNITED STATES, vol. 28, no. 6, 12 May 2017 (2017-05-12), pages 1-15, XP036234103, ISSN: 0957-4530, DOI: 10.1007/S10856-017-5898-3 [retrieved on 2017-05-12] * abstract * * 2.2 * | 1-6,8-13 | INV. A61L15/32 A61L15/44 |
| X | CN 102 499 996 A (WUXI ZHONGKE GUANGYUAN BIOLOG MATERIAL CO LTD) 20 June 2012 (2012-06-20) * abstract * * paragraph [0013] - paragraph [0029] * | 1-3,5-15 | |
| | | | TECHNICAL FIELDS SEARCHED (IPC) |
| | | | A61L |

The present search report has been drawn up for all claims

| Place of search | Date of completion of the search | Examiner |
|---|---|---|
| The Hague | 16 March 2021 | Fort, Marianne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 20 0785

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2021

| Patent document cited in search report | Publication date | Patent family member(s) | Publication date |
|---|---|---|---|
| CN 102499996 A | 20-06-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8728498 B
- KR 20080104932 A
- US 9744262 B
- US 7041868 B
- CN 107397973

### Non-patent literature cited in the description

- **WEI, Q ; XU, F. ; XU, X. et al.** The multifunctional wound dressing with core-shell structured fibers prepared by coaxial electrospinning. *Front. Mater. Sci.,* 2016, vol. 10, 113-121, https://doi.org/10.1007/s11706-016-0339-7
- **MULHOLLAND EJ.** Electrospun Biomaterials in the Treatment and Prevention of Scars in Skin Wound Healing. *Front Bioeng Biotechnol. 2020,* 03 June 2020, vol. 8, 481
- **SAH H ; THOMA LA ; DESU HR ; SAH E ; WOOD GC.** Concepts and practices used to develop functional PLGA-based nanoparticulate systems. *Int J Nanomedicine,* 2013, vol. 8, 747-765
- **SNEJDROVA E ; PODZIMEK S ; MARTISKA J ; HOLAS O ; DITTRICH M.** Branched PLGA derivatives with tailored drug delivery properties. *Acta Pharm.,* 2020, vol. 70 (1), 63-75
- **KHIL et al.** Electrospun nanofibrous polyurethane membrane as wound dressing. *J Biomed Mater Res B Appl Biomater.,* 2003, vol. 67 (2), 675-679
- **CHOU ; WOODROW.** Relationships between mechanical properties and drug release from electrospun fibers of PCL and PLGA blends. *J Mech Behav Biomed Mater,* 2017, vol. 65, 724-733
- **J. POSNETT ; F. GOTTRUP ; H. LUNDGREN ; G. SAAL.** *Journal of Wound Care,* 2009, vol. 18, 154
- **S. SCHREML ; R.-M. SZEIMIES ; L. PRANTL ; M. LANDTHALER ; P. BABILAS.** *Journal of the American Academy of Dermatology,* 2010, vol. 63 (866
- **S. R. NUSSBAUM ; M. J. CARTER ; C. E. FIFE ; J. DAVANZO ; R. HAUGHT ; M. NUSGART ; D. CARTWRIGHT.** *Value in Health,* 2018, vol. 21, 27
- **B. A. MAST ; G. S. SCHULTZ.** *Wound Rep. Reg.,* 1996, vol. 4, 411
- **S. DAS ; G. SINGH ; M. MAJID ; M. B. SHERMAN ; S. MUKHOPADHYAY ; C. S. WRIGHT ; P. E. MARTIN ; A. K. DUNN ; A. B. BAKER.** *Adv. Healthcare Mater.,* 2016, vol. 5, 2248
- **M. HRYNYK ; R. J. NEUFELD.** *Burns,* 2014, vol. 40, 1433
- **A. OLIVEIRA ; S. SIMÕES ; A. ASCENSO ; C. P. REIS.** *Journal of Dermatological Treatment,* 2020, 1
- **S. BARRIENTOS ; H. BREM ; O. STOJADINOVIC ; M. TOMIC-CANIC.** *Wound Repair Regen,* 2014, vol. 22, 569
- **B. D. ALMQUIST ; S. A. CASTLEBERRY ; J. B. SUN ; A. Y. LU ; P. T. HAMMOND.** *Adv. Healthcare Mater.,* 2015, vol. 4, 2090
- **G. GAINZA ; S. VILLULLAS ; J. L. PEDRAZ ; R. M. HERNANDEZ ; M. IGARTUA.** *Nanomedicine: Nanotechnology, Biology and Medicine,* 2015, vol. 11, 1551
- **G. ANDREAS ; W. J. H.** *Angewandte Chemie International Edition,* 2007, vol. 46, 5670
- **M. LIU ; X.-P. DUAN ; Y.-M. LI ; D.-P. YANG ; Y.-Z. LONG.** *Materials Science and Engineering: C,* 2017, vol. 76, 1413
- **F. CROISIER ; G. ATANASOVA ; Y. POUMAY ; C. JÉRÔME.** *Adv. Healthcare Mater.,* 2014, vol. 3, 2032
- **T. K. HUNT ; H. W. HOPF.** *Surgical Clinics of North America,* 1997, vol. 77, 587
- **S. DHIVYA ; V. V. PADMA ; E. SANTHINI.** *BioMed,* 2015, vol. 5, 22
- **D. HAN ; A. J. STECKL.** *ChemPlusChem,* 2019, vol. 84, 1453
- **T. DECKER ; M.-L. LOHMANN-MATTHES.** *Journal of Immunological Methods,* 1988, vol. 115, 61
- **C.-C. LIANG ; A. Y. PARK ; J.-L. GUAN.** *Nat Protoc,* 2007, vol. 2, 329
- **J. E. N. JONKMAN ; J. A. CATHCART ; F. XU ; M. E. BARTOLINI ; J. E. AMON ; K. M. STEVENS ; P. COLARUSSO.** *Cell Adhesion & Migration,* 2014, vol. 8, 440
- **A. NAJJAR ; M. ALAWI ; N. ABUHESHMEH ; A. SALLAM.** *Advances in Pharmaceutics 2014,* vol. 2014, 1
- **A. K. ATTRI ; C. FERNÁNDEZ ; A. P. MINTON.** *Biophysical Chemistry,* 2010, vol. 148, 28